(19) Europäisches Patentamt
European Patent Office
Office européen des brevets

(11) EP 0 634 137 B1

(12) **EUROPEAN PATENT SPECIFICATION**

(45) Date of publication and mention
of the grant of the patent:
**12.02.1997 Bulletin 1997/07**

(51) Int. Cl.$^6$: **A61B 5/0472**, A61B 5/0402,
H03H 17/00

(21) Application number: **94109663.8**

(22) Date of filing: **22.06.1994**

(54) **Device for eliminating ringings in filtered ECG-signals**

Vorrichtung zur Beseitigung von Nachschwingungen in gefilterten EKG-Signalen

Dispositif pour éliminer les sonores dans des signaux filtrés d'ECG

(84) Designated Contracting States:
**AT BE CH DE DK ES FR GB IT LI NL SE**

(30) Priority: **16.07.1993 SE 9302433**

(43) Date of publication of application:
**18.01.1995 Bulletin 1995/03**

(73) Proprietor: **Siemens-Elema AB**
**171 95 Solna 1 (SE)**

(72) Inventors:
- **Karlsson, Peter**
  **S-133 37 Stockholm (SE)**
- **Ohlsson, Thomas**
  **S-165 77 Hässelby (SE)**

(56) References cited:
WO-A-93/05573          US-A- 4 422 459
US-A- 5 139 027        US-A- 5 211 179

- **Journal of electrocardiology, Volume 23, 1990,
  Lynn Y. Shelton et al, "Detection of Late
  Potentials by Adaptive Filtering pp 138-143"**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 634 137 B1

## Description

The present invention relates to a device for eliminating ringings in filtered ECG-signals, comprising an A/D-converter for converting analog input signals into a series of digital values and a filter unit connected to the output of the A/D-converter.

Different kinds of signal filtration are required for the analysis of ECG-signals.

The ECG-signal recorded between two electrodes on the patient's body, e.g. on the arms and legs, also contains a DC potential, in addition to the ECG-signal, which may be much larger than the ECG-signal. These DC potential or low-frequency signals vary because of relative movements of the body and the electrode as a result of e.g. the patient's breathing movements.

Two-pass filtering is one way to remove these DC signals and low-frequency signals from the ECG-signal. A filter with a linear phase is then provided by both forward and backward filtration of the signal with a filter with a non-linear phase, see e.g. GB patent 1 556 512, which describes this filtration with analog filters. A linear phase is desirable at the filtration, since phase distortion is then eliminated and with that a minor distortion of the ECG-signal's morphology results. However, the disadvantage of a filter with a linear phase is that the ringing problem is not completely eliminated. Ringings, whose morphology and duration depend on the filter's transmission function, will persist. A so-called FIR-filter can also be used for realizing a filter with a linear phase, but the steepness of the filter's slope must be sacrificed if the filter is to be implemented in a device with limited memory or limited computational capacity.

The presence of high-frequency signals with small amplitudes on the microvolt level at the end of the QRS-complex in post-infarction patients has been shown to be a good indication of an increased risk of future life-menacing ventricular arrhythmias, see European Heart Journal (1991), 12, pp. 473 - 480. High-pass filtration is needed in order to identify and analyze these late potentials. One problem is then the ringing induced by the QRS signal itself and which readily drowns out any late potentials. Several proposals to solutions of this problem have been presented. In Link and Trahms, "Highpass Filters for Detecting Late Potentials", Proc. Computers in Cardiology 1992, pp. 159 - 162, the use of a non-recursive, monotone, binomial, high-pass filter for detecting late potentials is proposed.

In US patent 4 422 459 is described a technic for disclosing the presence or absence of a time segment containing high-frequency signals in the latter part of a patient's QRS-complex and for measuring the magnitude of this segment. The measured analog ECG-signals are then converted into digital signals, and normal QRS-signals are averaged over some hundred or so beats in order to obtain a relatively noise-less complex. The averaged complex is then subjected to high-pass filtration in a 4th order Butterworth-filter. The filtration is performed in the forward direction until the start of QRS + 40 ms and in the backward direction from the end up to the same point. The two signals are then combined to form a resultant signal in which ringings are avoided immediately before and after the QRS at the expense of an "erroneous" signal in the QRS itself. The filtered signal is then subjected to measurements to ascertain presence or absence of so-called late potentials. The disadvantage of this technic is that the filtered curve is discontinuous, and the QRS-complex has an erroneous energy content.

In US patent 4 458 691 is described a system for high-pass forward filtration of ECG-signals for detection of late potentials, an adaptive high-pass filter for selective filtration of different segments of the QRS-complex being used to solve the problem of ringing. This technic also has several disadvantages. Ringing is thus not completely eliminated, and to reduce the ringing effect as much as possible short FIR-filters with a linear phase are used, yielding poorer frequency splitting.

In US patent 4 458 692 is described another method for detecting late potential through forward filtration of the ECG-signal, regulation of filter gain depending on the magnitude of the input signal then being employed in trying to solve the problem of ringing. The signal controlling the gain can be the output signal from a filter using the QRS-signal as input signal, and e.g. the R-spike's impact on the output signal and, thus, on the ringing occurring after the R spike, can be limited if the gain in the filter is limited when the input signal rises above a defined level. This method also has the disadvantage of failing to eliminate all ringings, and a steep filter slope must be sacrificed.

US patent 4 492 235 also relates to detection of late potentials through forward filtration of the QRS-signal, an adaptive high-pass filtration being employed to overcome the problems of ringing.

In US patent 5 025 794 is described a further method for forward filtration of late potentials in which sampled values of the QRS-signal are subjected to both forward and backward filtration, whereupon the filtered signals are added together, and the summation signal is smoothed to yield signal components corresponding to the late potentials. The method described in this patent is complicated.

The object of the present invention is to provide a device for eliminating ringings in filtered ECG-signals, in which the above discussed disadvantages in the prior art are eliminated.

This purpose is achieved with a device with the features set forth in claim 1.

The device according to the invention thus comprises an allpass-filter with a phase shift which exactly corresponds to twice the phase shift of the original filter which has given rise to the ringings which is to be eliminated. The allpass-filter is applied to the part of the signal, which is to be analyzed, in a time-reversed direction in relation to the original filtration direction. In this way ringings are eliminated in a for the applications in question highly advantageous manner.

Thus, the device according to the invention makes possible simple detection of late potentials after the signal has been filtered in the forward direction with a recursive high-pass filter and without any restrictions on the filter's steepness. The device according to the invention can also be advantageously used for delimiting and classifying the form of the QRS-complex and for determining the ST level.

It should be noted that the result of filtering in the recursive filter and the subsequent time-reserved allpass-filtration is not a filtration with a linear phase but a filtration with a phase response negated in relation to the phase response of the recursive filter. This is an optimum characteristic for complete elimination of ringings in the applications in question. The total phase shift in the device according to the invention is thus the same as the phase shift occurring in backward filtration of the signal with the recursive IIR-filter. Thus, ringings are "shifted" away from the area of interest according to the same principle as in backward filtration, although the original filtration was performed in the forward direction in the conventional manner.

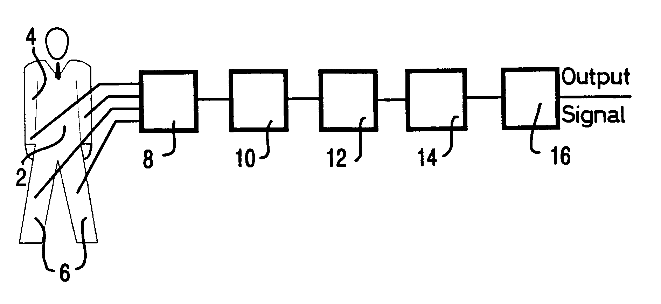

One exemplified embodiment of the device according to the invention will now be described in greater detail with reference to the enclosed FIGURE which shows an equipment in block diagram form for recording ECG's with the device according to the invention.

The ECG is recorded as the potential between electrodes on the patient's 2 body, e.g. on her/his arms 4 and legs 6. This recording as well as analog signal conditioning, takes place in the unit 8. In this unit 8, the large DC components which the ECG-signal contains, can a.o. be removed to make possible measuring of the ECG itself around the zero line. In this way the dynamic range and the resolution required by the subsequent A/D-converter 10 are reduced.

The output of the A/D-converter 10 is connected to an averager 12 to form an average signal over a number of QRS-complexes, said average signal then being subjected to forward filtering with a recursive IIR-filter 14, preferably a Butterworth-filter.

After the filter 14, there is a phase-shifting allpass-filter 16 in which the latter part of the averaged complex is filtered in a time-reversed direction. The ringing caused by the IIR-filter 14 has been eliminated from the output signal from the allpass-filter 16, so more accurate measurement and analysis of this output signal becomes possible.

The transfer function of the Butterworth-filter 14 can be written:

$$H_B(Z) = \frac{b_1 + b_2 Z^{-1} + b_3 Z^{-2} + b_4 Z^{-3} + b_5 Z^{-4}}{1 + a_2 Z^{-1} + a_3 Z^{-2} + a_4 Z^{-3} + a_5 Z^{-4}} = \frac{N(Z)}{D(Z)}$$

The allpass-filter 16 is constructed by replacing the zero points of the Butterworth-filter 14 with new zero points consisting of the poles of the filter reflected in the unity circle. The transfer function of the allpass-filter 16 then becomes:

$$H_A(Z) = \frac{Z^{-4} D(Z^{-1})}{D(Z)} = \frac{a_5 + a_4 Z^{-1} + a_3 Z^{-2} + a_2 Z^{-3} + Z^{-4}}{1 + a_2 Z^{-1} + a_3 Z^{-2} + a_4 Z^{-3} + a_5 Z^{-4}}$$

The allpass-filter 16 becomes a Butterworth-filter with the same poles as the filter it is to compensate for, and the zero points are thus obtained by reflecting the poles in the unity circle in the Z-plane.

The phase shift of the allpass-filter 16 then becomes

$$\Phi_A(\omega) = -4\omega + \Phi_D(\omega) - \Phi_D(-\omega) = 2\Phi_D(\omega) - 4\omega$$

$\Phi_D(\omega)$ = Phase shift for a filter with only zero points, i.e.

$$H(Z) = \frac{1}{D(Z)} \text{ and}$$

$$\Phi_D(-\omega) = -\Phi_D(\omega)$$

The phase shift for the Butterworth-filter is

$$\Phi_D(-\omega) = -2\omega + \Phi_D(\omega)$$

The total phase shift for the Butterworth-filter in a cascade with the allpass-filter in the device according to the invention accordingly becomes the following, since allpass-filtration takes place in the backward direction:

$$\Phi_{total}(\omega) = \Phi_B(\omega) - \Phi_A(\omega) = -\Phi_D(\omega) + 2\omega = -\Phi_B(\omega)$$

The phase shift after the device according to the invention is thus the same as the phase shift obtained in backward filtering of the signal with the Butterworth-filter. So the ringings are "moved" away from the area of interest, although the original filtration was performed in forward direction in the conventional way.

The design of the allpass-filter starts from the Butterworth-filter which produces the ringings to be eliminated and replaces its zero points with new zero points obtained by reflecting the poles of the filter in the unity circle in the Z-plane. It is assumed that all the zero points of the IIR-filter are situated on the unity circle in the Z-plane. The phase shift for this allpass-filter becomes

$$\Phi_A(\omega) = 2^*\Phi_H(\omega)+(n_H-n_A) \cdot \omega$$

$\Phi_H(\omega)$ = the phase shift of the Butterworth-filter
$n_H$ = the number of zero points in the Butterworth-filter
$n_A$ = the number of zero points in the allpass-filter

Since the Butterworth-filter has as many zero points as poles, the second term becomes equal to zero. Otherwise, compensation must be made for a constant delay after the allpass-filtration. It should be noted that the result of the filtration in the Butterworth-filter and the time-reversed allpass-filtration will not be a filtration with a linear phase but a filtration with a phase response which is negated in relation to the phase response of the Butterworth-filter, whereby optimum elimination of ringings is achieved for the applications in question.

## Claims

1. Device for eliminating ringings in filtered ECG-signals, comprising an A/D-converter for converting analog input signals into a series of digital values and a filter unit connected to the output of the A/D-converter, **characterized in** that the filter unit comprises a recursive IIR-filter and a subsequent allpass-filter with a phase shift which is equal to twice the phase shift of the IIR-filter, the allpass-filtration being performed time-reversed in relation to the IIR-filtration.

2. Device according to claim 1, **characterized in** that an averager is arranged between the A/D-converter and the filter unit in order to form a signal average over a number of QRS-complexes before the filtration.

3. Device according to claim 1 or 2, **characterized in** that the filter unit is arranged so only the latter part of the QRS-complex is filtered in the allpass-filter.

4. Device according to claim 1 or 2, **characterized in** that the filter unit is so arranged that only the earlier part of the QRS-complex is filtered in the allpass-filter.

5. Device according to any of claims 1-4 comprising said allpass-filter with a phase shift which is equal to twice the phase shift of the IIR-filter the zero points of which are situated on a unity circle in the Z-plane, **characterized in** that the allpass-filter is produced in that the zero points of the IIR-filter are replaced by new zero points obtained by reflecting the poles of the IIR-filter in the unity circle in the Z-plane.

## Patentansprüche

1. Vorrichtung zum Beseitigen des Nachschwingens in gefilterten EKG-Signalen mit einem A/D-Wandler zum Umwandeln analoger Eingangssignale in eine Serie digitaler Werte und mit einer Filtereinheit, die mit dem Ausgang des A/D-Wandlers verbunden ist, **dadurch gekennzeichnet**, daß die Filtereinheit ein rekursives IIR-Filter und ein nachfolgendes Allpaßfilter mit einer Phasenverschiebung aufweist, die gleich zweimal der Phasenverschiebung des IIR-Filters ist, wobei die Allpaßfilterung in Relation zu der IIR-Filterung zeitlich rückwärts durchgeführt wird.

2. Vorrichtung nach Anspruch 1, **dadurch gekennzeichnet**, daß ein Mittelwertbildner zwischen dem A/D-Wandler und der Filtereinheit angeordnet ist, um vor der Filterung einen Signalmittelwert über eine Anzahl von QRS-Komplexen zu bilden.

3. Vorrichtung nach Anspruch 1 oder 2, **dadurch gekennzeichnet**, daß die Filtereinheit so ausgebildet ist, daß nur der letztere Teil des QRS-Komplexes in dem Allpaßfilter filtriert wird.

4. Vorrichtung nach Anspruch 1 oder 2, **dadurch gekennzeichnet**, daß die Filtereinheit so ausgebildet ist, daß nur der frühere Teil des QRS-Komplexes in dem Allpaßfilter filtriert wird.

5. Vorrichtung nach einem der Ansprüche 1-4 mit dem Allpaßfilter mit einer Phasenverschiebung, die gleich zweimal der Phasenverschiebung des IIR-Filters ist, dessen Nullpunkte auf einem Einheitskreis in der Z-Ebene liegen, **dadurch gekennzeichnet**, daß das Allpaßfilter dadurch hergestellt wird, daß die Nullpunkte des IIR-Filters durch neue Nullpunkte ersetzt werden, die durch Reflektieren der Pole des IIR-Filters an dem Einheitskreis in der Z-Ebene erhalten werden.

**Revendications**

1. Dispositif destiné à éliminer les ondulations dans des signaux d'ECG filtrés, comprenant un convertisseur A/N destiné à convertir des signaux d'entrée analogiques en une série de valeurs numériques, et une unité de filtrage connectée à la sortie du convertisseur A/N, caractérisé en ce que l'unité de filtrage comprend un filtre RII récursif et un filtre passe-tout situé en aval ayant un déphasage qui est égal à deux fois le déphasage du filtre RII, le filtrage passe-tout étant effectué dans un sens inversé dans le temps par rapport au filtrage RII.

2. Dispositif selon la revendication 1, caractérisé en ce qu'un dispositif de calcul de moyenne est disposé entre le convertisseur A/N et l'unité de filtrage pour élaborer une moyenne du signal sur un certain nombre de complexes QRS avant le filtrage.

3. Dispositif selon la revendication 1 ou 2, caractérisé en ce que l'unité de filtrage est conçue pour ne filtrer que la dernière partie du complexe QRS dans le filtre passe-tout.

4. Dispositif selon la revendication 1 ou 2, caractérisé en ce que l'unité de filtrage est conçue pour ne filtrer que la première partie du complexe QRS dans le filtre passe-tout.

5. Dispositif selon l'une quelconque des revendications 1-4, comprenant le filtre passe-tout, celui-ci ayant un déphasage qui est égal à deux fois le déphasage du filtre RII dont les zéros sont situés sur un cercle unité dans le plan Z, caractérisé en ce que, dans le filtre passe-tout produit, les zéros du filtre RII sont remplacés par de nouveaux zéros obtenus en réfléchissant les pôles du filtre RII dans le cercle unité représenté dans le plan Z.

Output
Signal

4

2

6

8  10  12  14  16